# EUROPÄISCHE PATENTSCHRIFT

(11) **EP 2 121 084 B1**
(45) Veröffentlichungstag und Bekanntmachung des Hinweises auf die Patenterteilung: **06.03.2019**
(21) Anmeldenummer: 08707085.0
(22) Anmeldetag: 17.01.2008
(51) Int. Cl.: A61M 5/20, A61M 5/315

(54) **EINWEGINJEKTOR MIT MINDESTENS EINEM ZENTRALEN ZUGSTAB**
DISPOSABLE INJECTOR WITH AT LEAST ONE CENTRAL TRACTION ROD
INJECTEUR À USAGE UNIQUE COMPRENANT AU MOINS UNE TIGE DE TRACTION CENTRALE

(30) Priorität: 16.02.2007 DE 102007008369
(43) Veröffentlichungstag der Anmeldung: 25.11.2009
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Thämer, Wolfgang
(86) Internationale Anmeldenummer: PCT/EP2008/000319
(87) Internationale Veröffentlichungsnummer: WO 2008/098656

(56) Entgegenhaltungen:
- EP-A- 0 144 625
- WO-A-02/47746
- WO-A-95/03844
- WO-A-03/092771
- WO-A-2006/062997
- AT-U1- 7 347
- FR-A- 393 189
- US-A- 4 874 367

## Beschreibung

Die Erfindung betrifft einen Einweginjektor mit einem Gehäuse, in dem oder an dem - jeweils zumindest bereichsweise - mindestens ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet ist, wobei der Federenergiespeicher mindestens ein vorgespanntes Federelement umfasst, wobei zumindest ein Teil des Kolbenbetätigungsstempels zwischen dem Federenergiespeicher und dem Kolben der Zylinder-Kolben-Einheit positioniert ist und wobei der federbelastete Kolbenbetätigungsstempel einen Zugstab aufweist, der im Bereich seines hinteren Endes mindestens eine Abstützfläche hat.

Aus der EP 0 595 508 B1 ist u.a. ein derartiger Injektor bekannt. Er hat einen federvorgespannten Kolben, dessen rückwärtige Kolbenstange an ihrem freien Ende einen elastischen Bereich mit einer Taille aufweist. Im Bereich der Taille ist der Kolben kraft- und formschlüssig am Injektorgehäuse festgeklemmt. Dazu steckt im freien Ende der Kolbenstange ein Auslösestift, der die Taille und deren Randbereiche auf ihren maximalen Durchmesser hält. Durch ein Verschieben des Auslösestifts kann sich der Durchmesser des Kolbenstangenendbereichs durch die wirkenden Klemmkräfte verringern, so dass die Klemmwirkung der Kolbenstange gegenüber dem Gehäuse verloren geht. In der Folge bewegt sich der federvorgespannte Kolben.

Die EP 0 144 625 A zeigt einen Einweginjektor gemäß dem Oberbegriff des Anspruchs 1, dessen Zugstab eine Einschnürung aufweist. In der Einschnürung liegen zwei einander gegenüber positionierte Kugeln - bei gespanntem Nadelinjektor - an. Die Kugeln stützen sich gehäuseseitig an zwei Flügeln radial und an einer Stütze axial ab. Beim Auslösen des Nadelinjektors werden die Flügel axial so weit verschoben, dass die Kugeln unter der Wirkung von Zwangskräften radial soweit ausweichen können, dass der Zugstab sich in Auslöserichtung bewegen kann.

Aus der FR 393 189 A ist ein Injektor mit einer Nadelsicherung bekannt, deren Sperrelemente über eine Feder aufeinander zu gezogen werden.

In der US 4 874 367 A ist ein Injektor beschrieben, dessen Zugstab mittels eines auf Zug belasteten Hakens gehalten wird. Eine Feder sorgt dafür, dass das Hintergriffsteil des Hakens dauernd an dem Zugstab anliegt.

In der WO 95/03844 A blockiert ein federbelasteter Querschieber die Zugstange.

Die AT 7 347 U1 und die WO 2006/062997 A zeigen jeweils einen Injektor, der mehrere Zugstäbe aufweist, die sich nicht an gelenkigen oder elastischen Sperrelementen, sondern an einer steifen, ebenen Platte abstützen.

In der WO 02/47746 A ist ein höchst komplizierter Nadelinjektor offenbart, bei dem zum Auslösen zunächst eine Nadelschutzhülse in den Injektor hinein geschoben werden muss, um dann mittels eines rückseitigen Druckknopfs geschlitzte Laschen so weit aufzudehnen und zugleich einen Auslöser so weit vorwärts zu schieben, dass der Federspeicher ausgelöst wird.

Die WO 03/092771 beschreibt einen Injektor, bei dem ein Querschieber einen zugstangenartigen Längsschlitten blockiert.

Der vorliegenden Erfindung liegt daher die Problemstellung zugrunde, einen modular aufgebauten Einweginjektor zu entwickeln, der bei geringer Baugröße nur wenige Bauteile aufweist und bei einfacher Handhabung eine sichere Lagerung und Funktion gewährleistet.

Diese Problemstellung wird mit den Merkmalen des Hauptanspruchs gelöst. Dau ist das Gehäuse ein topfförmiger, unten offener Hohlkörper mit obenliegendem Zwischenboden. An der oder den Abstützflächen liegen am Gehäuse abgestützte Sperrelemente an, wobei die Sperrelemente entweder Winkelelemente sind, die im Bereich ihrer Knickstelle am Zwischenboden gelenkig gelagert sind oder wobei die Sperrelemente an einem ringartigen, auf dem Zwischenboden aufliegenden Bauteil befestigt sind oder wobei die Sperrelemente als biegeelastische Elemente am Zwischenboden angeformt sind, wobei die sperrende Lage der Sperrelemente durch ein in einer Sperrstellung positioniertes Auslöseelement gesichert ist. Das Auslöseelement hat eine Lösestellung, die ein Freigeben der Sperrelemente bewirkt.

Mit der Erfindung wird hier beispielsweise ein nadelfreier Einmalinjektor vorgestellt, dessen Kolbenbetätigungsstempel bei einem Auslösevorgang des Einweginjektors freigegeben wird. Dazu liegt zum Vorspannen und Halten des Federenergiespeichers mindestens ein Teil des Kolbenbetätigungsstempels mit mindestens einer umgriffartigen Kontur zumindest kraftschlüssig am Gehäuse oder einem am Gehäuse angeordneten Bauteil, z.B. einem Sperrelement, an. Diese Sperrelemente werden von einem Auslöseelement bis zum Gebrauch des Einweginjektors in ihrer Sperrposition gehalten. Zum Auslösen des Injektors werden die Sperrelemente freigegeben, so dass sich der Kolbenbetätigungsstempel - unter der Wirkung des Federenergiespeichers - parallel zur Mittellinie des Einweginjektors bewegen kann.

Weitere Einzelheiten der Erfindung ergeben sich aus den Unteransprüchen und den nachfolgenden Beschreibungen von einigen schematisch dargestellten Ausführungsbeispielen.
- Figur 1:: Einweginjektor mit einem zentralen Zugstab;
- Figur 2:: wie Figur 1, jedoch entsichert und betätigt;
- Figur 3:: wie Figur 2, jedoch nach dem Medikamentenausstoß;
- Figur 4:: Querschnitt zu Figur 1 im Bereich des Sicherungselementes;
- Figur 5:: Einweginjektor mit einem Zugstab, der eine kegelmantel- oder keilförmige Abstützfläche hat;
- Figur 6:: Ausschnittvergrößerung zu Figur 5;
- Figur 7:: wie Figur 5, jedoch entsichert und betätigt;
- Figur 8:: wie Figur 5, jedoch nach dem Medikamentenausstoß;
- Figur 9:: dimetrische Draufsicht auf das Gehäuse des Einweginjektors nach Figur 5;
- Figur 10:: Seitenansicht zum Einweginjektor nach Figur 5, jedoch vor der Benutzung;
- Figur 11:: Einweginjektor mit einem Zugstab, der eine torusteilmantelförmige Abstützfläche hat;
- Figur 12:: wie Figur 11, jedoch entsichert und betätigt;
- Figur 13:: wie Figur 11, jedoch nach dem Medikamentenausstoß;
- Figur 14:: dimetrische Draufsicht auf das Gehäuse des Einweginjektors nach Figur 11;
- Figur 15:: Seitenansicht zum Einweginjektor nach Figur 11, jedoch vor der Benutzung;
- Figur 16:: Einweginjektor nach Figur 5, jedoch mit vollständig geschlossenem Gehäuse;
- Figur 17:: wie Figur 16, jedoch entsichert und betätigt;
- Figur 18:: wie Figur 17, jedoch nach dem Medikamentenausstoß;
- Figur 19:: dimetrische Draufsicht auf das Gehäuse des Einweginjektors nach Figur 16;
- Figur 20:: Seitenansicht zum Einweginjektor nach Figur 16, jedoch vor der Benutzung.

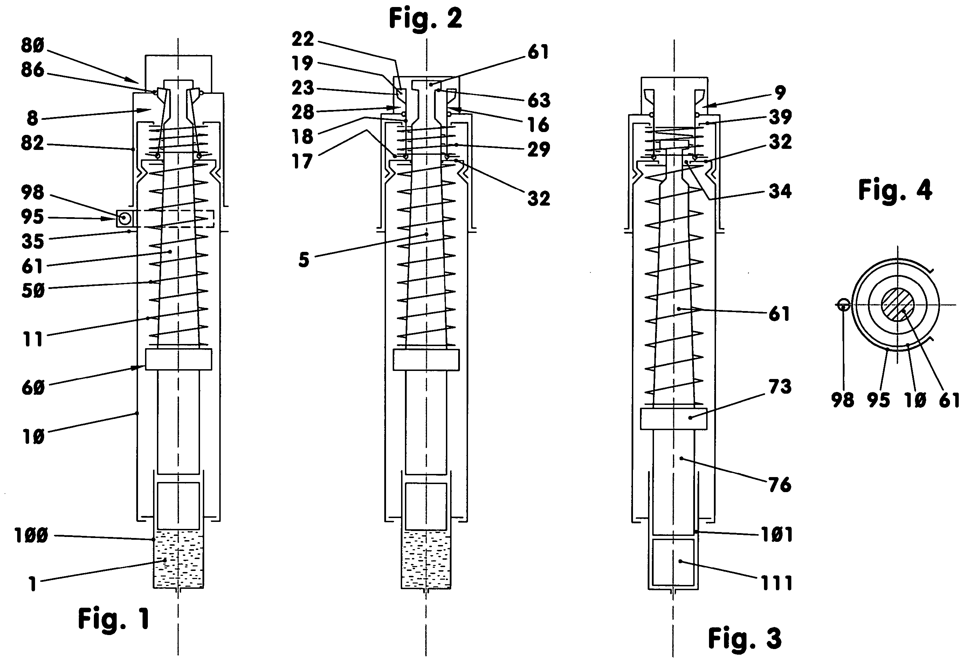

Die Figuren 1 bis 3 zeigen das vereinfachte Prinzip eines Einweginjektors mit einem dauergeladenen Federenergiespeicher. Der Einweginjektor besteht aus einem Gehäuse (10), einer z.B. befüllten Zylinder-Kolben-Einheit (100), einem Kolbenbetätigungsstempel (60) mit Zugstab (61) und einer Schraubendruckfeder (50) als Federenergiespeicher. Zudem sind am Gehäuse (10) ein Auslöseelement (82) und ein Sicherungselement (95) angeordnet.

Das Gehäuse ist ein topfförmiger, unten offener Hohlkörper mit obenliegendem Zwischenboden (32). Der Zwischenboden (32) hat z.B. eine mittige Öffnung (34), durch die nach Figur 1 der Zugstab (61) hindurch gesteckt ist. Der Zugstab (61) liegt mit mindestens einer Abstützfläche (63) an Sperrelementen (16) an, die auf dem Zwischenboden (32) z.B. beidseits des Zugstabes (61) gelagert sind, vgl. Figur 2.

Die Sperrelemente (16) sind nach den Figuren 1 bis 3 spezielle Winkelhebel, die im Bereich ihrer Knickstelle am Zwischenboden (32) gelenkig gelagert sind. Jedes Sperrelement (16) hat einen kurzen (17) und einen langen Hebelarm (18). Der lange Hebelarm (18) hat am oberen Ende ein Stützelement (19) mit einer oberen (22) und einer seitlichen Anlagefläche (23). Unterhalb des seitlich vorstehenden Stützelements (19) befindet sich eine Rücksprungkontur (28). Ihr Abstand zur Mittellinie (5) ist erheblich kürzer als der kürzeste Abstand zwischen der seitlichen Anlagefläche (23) und der Mittellinie (5).

Ggf. können die Sperrelemente (16) an einem ringartigen Bauteil befestigt sein. Dieses Bauteil liegt dann beispielsweise auf dem Zwischenboden (32) auf. Von ihm stehen die Sperrelemente (16) nach oben ab. Die zwischen je zwei Sperrelementen gelegenen Abschnitte des ringartigen Bauteils dienen u.a. als Torsionsfeder, die die Sperrelemente (16) aus einer Sperrstellung (8) in eine Lösestellung (9) bringt.

Die obere Anlagefläche (22) des einzelnen Sperrelements (16) kontaktiert die Abstützfläche (63) des Zugstabes (61). Die Anlagefläche (22) und die Abstützfläche (63) nehmen in der Sperrstellung (8) gegenüber der Mittellinie (5) jeweils zumindest annähernd den gleichen Winkel ein. Er liegt z.B. in einem Bereich von 10 bis 85 Winkelgraden, wobei hier die höheren Winkelgrade bevorzugt werden.

Die oberen Anlageflächen (22) und die Abstützflächen (63) sind in der Regel sphärisch gekrümmt und in der Sperrstellung (8) zumindest annähernd aneinander angepasst. Es sind jedoch auch Flächen (22) und (63) denkbar, die eben bzw. plan gestaltet sind.

Der kürzere Hebel (17) des einzelnen Sperrelements (16) steht seitlich vom langen Hebel (18) ab. Im Ausführungsbeispiel, vgl. Figur 2 und 3, ist der kurze Hebel (17) radial zur Mittellinie (5) hin orientiert. Auf dem kurzen Hebel (17) aller Sperrelemente (16) liegt eine Schraubendruckfeder (29) auf. Letztere stützt sich nach oben hin an einem Gehäuseumgriff (39) ab. Die Schraubendruckfeder (29) bewirkt ein Auseinanderschwenken der Sperrelemente (16). Dabei werden nach Figur 1 jeweils die seitlichen Anlageflächen (23) der Sperrelemente (16) gegen das Auslöseelement (82) gepresst.

Das Auslöseelement (82) ist eine Art kappenförmiger Druckknopf, der auf das obere Ende des Gehäuses (10) längsverschiebbar aufgesteckt ist. Es liegt dazu an der z.B. zylindermantelförmigen Außenfläche des Gehäuses (10) gleitgelagert an.

Von der Innenseite des Auslöseelementes (82) stehen Stützstege (86) z.B. radial nach innen ab. Die Stützstege (86), die auch Teil eines Ringes oder einer Lochscheibe sein können, haben Vorderkanten, an denen sich die seitlichen Abstützflächen (23) der Sperrelemente (16) abstützen.

Die Art der Auslösung ist nicht auf die hier beschriebene Variante beschränkt. Anstelle des längsverschiebbaren, auf die Sperrelemente (16) wirkenden Auslöseelements (82) kann z.B. u.a. ein Exzentergetriebe, ein Schraubgetriebe oder ein Hebelgetriebe treten.

Das Auslöseelement (82) liegt nach Figur 1 an einem Sicherungselement (95) an. Letzteres stützt sich z.B. an einem Gehäusekragen (35) nach unten ab. Das Sicherungselement (95) ist beispielsweise ein elastischer omegaförmiger Bügel, der die Außenfläche des Gehäuses (10) auf einem Winkel von ca. 120 Winkelgraden federnd umschließt. Nach den Figuren 1 und 4 hat er auf der linken Seite einen Griff (98), über den er - zum Entsichern - seitlich abgezogen werden kann.

Im Gehäuse (10) ist der Kolbenbetätigungsstempel (60) angeordnet. Er ist in drei Bereiche aufgeteilt. Der untere Bereich ist der Kolbenschieber (76). Sein Durchmesser ist etwas kleiner als der Innendurchmesser des Zylinders (101) der Zylinder-Kolben-Einheit (100). Die untere Stirnfläche des Kolbenschiebers (76) wirkt direkt auf den Kolben (111).

Der Kolbenschieber (76) kann selbstverständlich auch als separates Bauteil ausgeführt sein. Hierzu ist er dann an der Innenwandung des Gehäuses (10) geführt.

Der mittlere Bereich ist der Stempelteller (73). Der Stempelteller (73) ist eine flache, zumindest bereichsweise zylindrische Scheibe, deren Außendurchmesser einige Zehntel Millimeter kleiner ist als der Innendurchmesser des Gehäuses (10) im Mantelbereich (31). Der obere Bereich ist der Zugstab (61).

Im unteren Teil des Gehäuses (10) ist die Zylinder-Kolben-Einheit (100) befestigt. Die Zylinder-Kolben-Einheit (100) besteht hier aus einem mit einer Injektionslösung (1) befüllten Zylinder (101), in dem ein Kolben (111) in der hinteren Position sitzt. Oberhalb des Kolbens (111) ist im Gehäuse (10) der Kolbenbetätigungsstempel (60) z.B. so angeordnet, dass er den Kolben zwar nicht berührt, jedoch mit seinem unteren Ende im oberen Bereich des Zylinders (101) seitlich geführt wird.

Zwischen dem Stempelteller (73) und dem obenliegenden Boden (32) des Gehäuses (10) sitzt vorgespannt die Schraubendruckfeder (50).

Zum Betätigen des Einweginjektors wird zunächst das Sicherungselement (95) seitlich abgezogen, die Düsenbohrung (106) des Einweginjektors auf der Injektionsstelle positioniert und anschließend, z.B. mit einem Finger der den Einweginjektor tragenden Hand, das Auslöseelement (82) gedrückt. Die Stützstege (86) des Auslöseelements (82) gleiten hierbei solange an den seitlichen Anlageflächen (23) der Sperrelemente (16) entlang, bis sie in den Bereich der Rücksprungkontur (28) gelangen. Wenn die Stützstege (86) die Rücksprungkontur (28) erreicht haben, federn sie unter der Wirkung der Schraubendruckfeder (29) auf, d.h. die Sperrelemente (16) schwenken nach außen, also weg von der Mittellinie (5).

Dieses Wegschwenken wird unterstützt durch die Anlagebedingungen im Bereich der Abstützfläche (63) und der oberen Anlagefläche (22). Wegen der räumlichen Neigung dieser Flächen (22, 63) wirken aufgrund des Federenergiespeichers (50) auf die Sperrelemente (16) permanent Querkräfte, die diese nach außen drücken.

Durch das Wegschwenken der Sperrelemente (16) wird der Zugstab (61) freigegeben. Er kann nun ungehindert durch die Bohrung (34) nach unten schnellen.

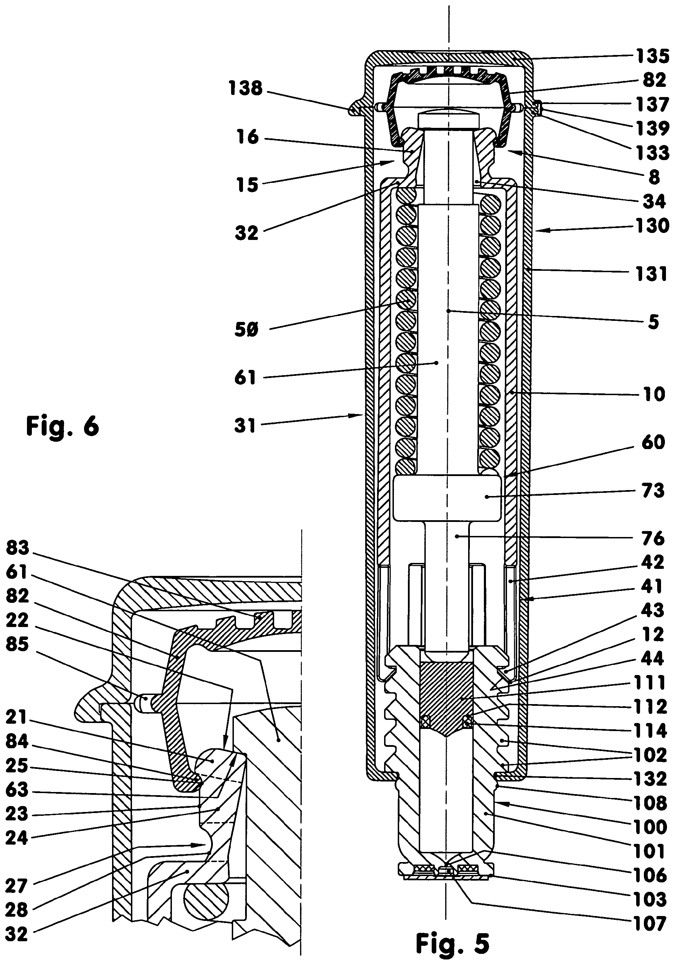

Die Figuren 5 bis 10 zeigen eine Ausführungsform des in den Figuren 1 bis 4 beschriebenen Prinzips. Hier ist das tragende Bauteil das Gehäuse (10). Es hat eine weitgehend rohrförmige Gestalt und ist in drei Funktionsbereiche (15, 31, 41) aufgeteilt. Nach den Figuren 5 und 6 ist der obere Bereich der Auslösebereich (15). An ihn schließt sich der Mantelbereich (31) an. Zwischen beiden Bereichen ist ein Boden (32) angeordnet. Der Boden (32) hat eine zentrale Ausnehmung (34).

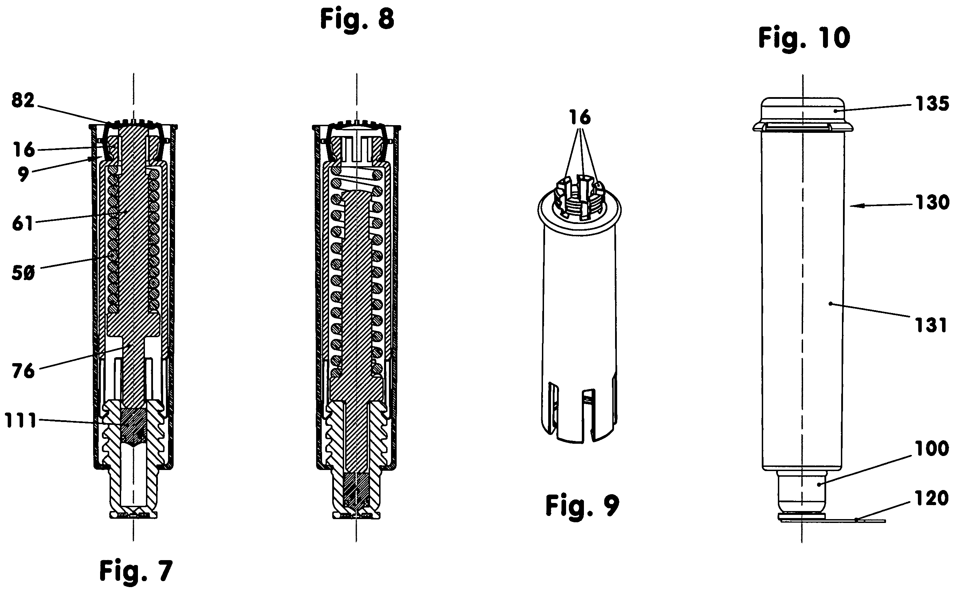

Im Auslösebereich (15) des Gehäuses (10) sind auf der Oberseite des Bodens (32) z.B. sechs angeformte Sperrelemente (16) angeordnet, vgl. auch die dimetrische Ansicht nach Figur 9. In Figur 5 sind zwei einander gegenüberliegende Sperrelemente (16) im Längsschnitt dargestellt. Das einzelne Sperrelement (16) ist in drei Bereiche unterteilt. Der erste Bereich ist der Anlagebereich (21), vgl. Figur 6. Letzterer kontaktiert mit der Anlagefläche (22) die Abstützfläche (63) des Zugstabes (61). Gleichzeitig liegt seine obere Innenkante ggf. am hier zylindrischen, taillierten Schaft des Zugstabes (61) - unterhalb der Abstützfläche (63) - an. Ggf. haben die Sperrelemente (16) zumindest im Bereich der Anlageflächen (22) eine keramische Panzerung.

Der zweite Bereich ist der Haltebereich (24) der Sperrelemente (16). Er ist vom Anlagebereich (21) durch eine Kerbe (25) getrennt. In der Kerbe (25) liegt der untere abstützende Rand (84) eines Auslöseelements (82) in der Sperrstellung (8) verrutschsicher an. Unterhalb der Kerbe (25) befindet sich die seitliche Anlagefläche (23), zu der auch die Kerbe (25) gezählt wird.

Über den dritten Bereich, den sog. Rücksprungbereich (27) ist das einzelne Sperrelement (16) mit dem Gehäuse (10) verbunden. Der Rücksprungbereich (27) weist eine Rücksprungkerbe (28) auf. In letztere ragt der Rand (84) des Auslöseelements (82) in der Lösestellung (9) hinein, vgl. Figuren 7 und 8.

Im unteren Bereich des Gehäuses (10) befindet sich der Fixierbereich (41) zur Aufnahme der einbaubaren Zylinder-Kolben-Einheit (100). Der Fixierbereich (41) umfasst z.B. sechs Federhaken (42), die jeweils in einer nach innen gerichteten Hakenspitze (43) enden. Die Hakenspitzen (43) haben zur unteren Gehäusestirnseite (12) hin eine sich über die gesamte Hakenstärke erstreckende Anfasung (44). Die Länge und die Federrate der Federhaken (42) ist so dimensioniert, dass die für die Funktion des Einweginjektors erforderlichen Einbauten (50, 100) ohne plastische Verformung der Federhaken (42) eingebaut werden können.

Einer dieser Einbauten ist die Zylinder-Kolben-Einheit (100), vgl. Figur 5. Sie besteht aus einem Zylinder (101) und einem Kolben (111). Der Zylinder (101) ist ein z.B. dickwandiger Topf, dessen ggf. zylindrische Außenwandung beispielsweise fünf umlaufende Rastrippen (102) trägt. Die Summe der Rastrippen (102) hat im Querschnitt z.B. ein Sägezahnprofil, wobei die Teilung zwischen den zahnartigen Rastrippen (102) äquidistant ist. Der maximale Durchmesser der Rastrippen (102) ist geringfügig kleiner als der Innendurchmesser des Gehäuses (10) im Fixierbereich (41). Der Durchmesser der zwischen benachbarten Rastrippen (102) liegenden Bereiche entspricht dem minimalen Durchmesser des Gehäuses (10) im Bereich der Hakenspitzen (43).

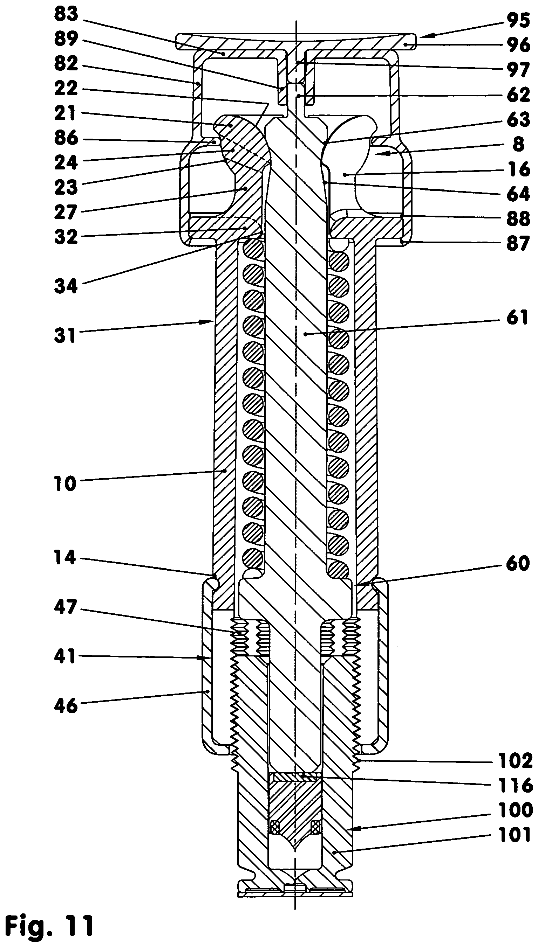

In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In die rückseitige Stirnfläche des Kolbens (111) ist ggf. eine z.B. zylindrische Metallplatte (116) eingelassen, vgl. Figur 11.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie mündet in einer zylindrischen Ausnehmung (107) der bodenseitigen, äußeren Stirnfläche (103) des Zylinders (101).

Zwischen dem Kolben (111) und dem Auslösebereich (15) ist der Federenergiespeicher (50) bzw. die Antriebseinheit des Einweginjektors angeordnet. Der Federenergiespeicher (50) ist eine Schraubendruckfeder, die auf einem Kolbenbetätigungsstempel (60) mit einem Zugstab (61) angeordnet ist. Mittels des Zugstabs (61) stützt sich der federkraftbelastete Kolbenbetätigungsstempel (60) an den Sperrelementen (16) des Gehäuses (10) ab.

Der Kolbenbetätigungsstempel (60) ist ebenfalls in drei Bereiche unterteilt. Der untere Bereich ist der Kolbenschieber (76), der mittlere Bereich ist der das Federelement (50) abstützende Stempelteller (73) und der obere Bereich ist der Zugstab (61), vgl. auch Beschreibung zu den Figuren 1 bis 4.

Im Auslösebereich (15) sitzt das kappenförmige, druckknopfartige Auslöseelement (82), vgl. Figur 5 und 6 in seiner oberen Sperrstellung (8). Das Auslöseelement (82) hat einen außen gerillten Boden (83), einen nach innen gewölbten Rand (84) und einen umlaufenden, teilweise unterbrochen überstehenden Außenrand (85). Der Rand (84) sitzt - in Sperrstellung (8) - in den Kerben (25) der Sperrelemente (16).

Der Außenrand (85) dient als Führungshilfe gegenüber einem Umgehäuse (130), vgl. auch Figur 10. Ein solches Umgehäuse (130) besteht aus einem Unterteil (131) und einem Oberteil (135). Das z.B. größere Unterteil (131) umgibt nach Figur 5 das gesamte Gehäuse (10) und einen Teil der Zylinder-Kolben-Einheit (100). Der Unterteilboden hat eine Bohrung (132), durch die die Zylinder-Kolben-Einheit (100) nach unten herausragt. Hier ist das Unterteil (131) zwischen der untersten Rastrippe (102) und einer umlaufenden Erhebung (108) fixiert.

Oben endet das Unterteil (131), vgl. Figur 5, in einem verbreiterten, flanschartigen Rand (133). Auf diesem Rand (133) liegt das Oberteil (135) mit seinem z.B. ebenfalls flanschartigen Rand (137) auf. An diesem Rand (137) ist beispielsweise ein Umgriff (138) und ein Filmgelenk (139) angeordnet. Das Filmgelenk (139) verbindet einteilig die Teile (131) und (135). Der Umgriff fixiert das Oberteil (135) auf dem Unterteil (131), indem es den Rand (133) elastisch umgreift. Das Oberteil (135) kann mit geringem Kraftaufwand von Hand vom Unterteil (131) weggeklappt werden.

Das Unterteil (131) ist ohne Filmgelenk (139) ein bezüglich der Mittellinie (5) rotationssymmetrisches Bauteil. Das Oberteil (135) kann an ihm z.B. auch mittels eines Schraub- oder Bayonettverschlusses befestigt sein. Zwischen dem Ober- (135) und dem Unterteil (131) kann auch eine Dichtung angeordnet oder z.B. an eines der Teile (131, 135) angeformt sein.

Das vollständig geschlossene Umgehäuse (130), dessen Unterteil (131) ggf. zusätzlich gegenüber der Zylinder-Kolben-Einheit (100) mittels einer Dichtung angedichtet ist, schützt den Einweginjektor auf einfache und sichere Weise. Es dient somit zugleich als Sicherungselement.

Die Figur 7 zeigt den Einweginjektor ohne Oberteil (135), also entsichert, mit niedergedrücktem Auslöseelement (82). Mit dem Niederdrücken des Auslöseelements (82) lösen sich die Sperrelemente (16) vom Zugstab (61) u.a. durch ein elastisches Zurückfedern und/oder durch plastisches Wegdrücken. Der Zugstab (61) kann nun unter der Wirkung des Federelements (50) nach unten schnellen und den Kolben (111) mittels des Kolbenschiebers (76) verschieben, vgl. Figur 8.

Die Figur 10 zeigt den noch unbetätigten Einweginjektor in der handelsüblichen Aufmachung. Das hier nicht sichtbare Gehäuse (10) und das Auslöseelement (82) sind vom Umgehäuse (130) gesichert umgeben. Die untere Stirnseite der Zylinder-Kolben-Einheit (100) ist mit Hilfe einer abreißbaren Klebeversiegelung (120) steril verschlossen.

In den Figuren 11 bis 15 wird eine Einweginjektor-Variante dargestellt, dessen z.B. großteils zylindrischer Zugstab (61) kraftschlüssig großflächig an beispielsweise vier Sperrelementen (16) anliegt.

Das Gehäuse (10) ist auch hier im Wesentlichen ein glattes Rohr mit einem obenliegenden, ebenen Boden (32), der zudem geringfügig radial über den Mantelbereich (31) übersteht. Im Boden (32) ist zum Durchführen des Kolbenbetätigungsstempels (60) eine zentrale Bohrung (34) eingearbeitet. Um die Bohrung herum sind besonders elastische Sperrelemente (16) angeordnet. Die Anzahl der Sperrelemente (16) liegt üblicherweise zwischen 3 und 12.

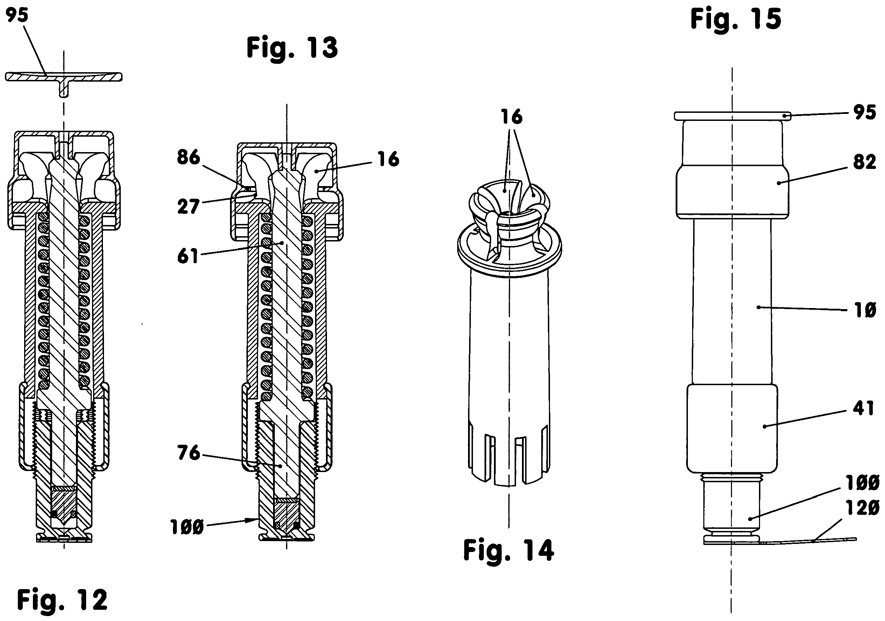

Das einzelne Sperrelement (16), von denen eines linksseitig in Figur 11 speziell im Schnitt dargestellt ist, hat - im Querschnitt betrachtet - im Anlagebereich (21) eine Anlagefläche (22) mit einer stetig gekrümmten Kontur. Die Kontur ist Teil einer Torusaußenfläche, vgl. auch Figur 14.

Der Zugstab (61) hat eine taillenartige Einschnürung, die bereichsweise die zugstabseitige Abstützfläche (63) darstellt. Letztere ist ein Teil einer Torusinnenfläche. Diese hat zumindest annähernd den gleichen Kreisquerschnittsradius und die gleichen Ringradien wie die Torusaußenfläche der Anlagefläche (22). Dadurch ergeben sich besonders große Kontaktflächen zwischen dem Zugstab (61) und den Sperrelementen (16). Die Abstützfläche (63) reicht hierbei bis zur engsten Stelle der Einschnürung.

Unterhalb dieser Abstützfläche (63) hat der Zugstab (61) z.B. eine rotationssymmetrische Übergangsfläche (64), die die Abstützfläche (63) mit der übrigen zylindrischen Außenfläche des Zugstabs (61) verbindet. Die Übergangsfläche (64) und die Abstützfläche (63) haben ausschließlich stetige Übergänge. Auch die anderen Flächenübergänge haben keine scharfkantigen Absätze, so dass auch bei einem kleinen Zugstabdurchmesser aufgrund geringer Kerbwirkungen eine große Zugkraft dauerhaft abgestützt werden kann.

Der Haltebereich (24) der Sperrelemente (16) ist der Bereich, in dem das Auslöseelement (82) mit seinem Stützsteg (86) anliegt.

Dazu ist das Auslöseelement (82) als eine auf dem Gehäuse (10) sitzende Kappe ausgebildet. Das Auslöseelement (82) umgreift - in der Sperrstellung (8) - den überstehenden Gehäuseboden (32). Hierfür weist es einen Umgriffsrand (87) und einen Steg (88) auf.

Der Stützsteg (86) befindet sich im mittleren Bereich der Kappe. Die Kappe (82) hat oben einen Boden (83) mit einem eingelassenen Rohrabschnitt (89).

Auf der Kappe (82) liegt ein Sicherungselement (95) auf, das aus einer tellerförmigen Scheibe (96) und einem angeformten Sperrzapfen (97) besteht. Der Sperrzapfen (97) steckt zentriert im Rohrabschnitt (89) der Kappe (82). Gleichzeitig liegt der Sperrzapfen (97) an einem zentralen Führungszapfen (62) des Zugstabs (61) an. In der Sperrstellung (8) steckt der Führungszapfen (62) zumindest bereichsweise im Rohrabschnitt (89) der Kappe (82).

Der Haltebereich (24) der Sperrelemente (16) liegt unterhalb des Stützsteges (86) eines in Sperrstellung (8) positionierten Auslöseelements (82). Die Kontur des Haltebereichs (24) ist Teil eines Torus, dessen Mittelpunkt auf der Mittellinie (5) in der Höhe des Stützsteges (86) liegt.

Der sich an den Haltebereich (24) anschließende Rücksprungbereich (27) hat in Sperrstellung (8) einen Außendurchmesser, der ca. 20 bis 25% kleiner ist als der größte Außendurchmesser des Haltebereiches (24).

Die vier Sperrelemente (16) dieser Variante haben im Haltebereich (24) große Stützquerschnitte. Die Summe der vier Querschnittsflächen beträgt im Rücksprungbereich (27) 60 bis 70% der Gesamtquerschnittsfläche des Gehäuses (10) unterhalb des Bodens (32).

Der untere Bereich des Mantelabschnitts (31) weist einen elastischen Fixierbereich (41) zur Lagerung einer Zylinder-Kolben-Einheit (100) auf. Er hat ein innen liegendes Rillenprofil (45), das durch Längsschlitze mehrfach unterbrochen ist. Mit dem Rillenprofil (45) wird die Zylinder-Kolben-Einheit (100) gehalten, die zumindest bereichsweise ein entsprechendes außen liegendes Rillenprofil (102) aufweist. Mittels des quer zur Mittellinie (5) orientierten Profils (45 ,102) kann die Zylinder-Kolben-Einheit (100) zur Variation des Zylindervolumens in verschiedenen Raststellungen im Gehäuse (10) positioniert werden. Zwei benachbarte Rastpositionen unterscheiden sich durch eine Zylindervolumendifferenz von 0,025 ml. Die einzelnen Rillen haben jeweils einen Zahnquerschnitt, wie sie z.B. bei Stahlpanzerrohrgewinden, oder bei Whitworth-Rohrgewinden anzutreffen sind. Die Zahnquerschnitte können aber auch von Trapez- , Sägen- oder Rohrgewinden übernommen sein.

Über den Fixierbereich (41) wird nach dem Einsetzen der Zylinder-Kolben-Einheit (100) eine die Position der Einheit (100) sichernde Klemmhülse (46) geschoben. Die Klemmhülse (46) verrastet dazu in einer Ringnut (14) des Gehäuses (10).

Wenn nach dem Abnehmen des Sicherungselements (95) und dem Abziehen der Schutzfolie (120), vgl. Figur 15, die Kappe (82) nach unten gedrückt wird, gleitet der Stützsteg (86) in den Rücksprungbereich (27) der Sperrelemente (16). Die Sperrelemente (16) federn mindestens soweit auseinander, dass der Zugstab (61) freigegeben wird, vgl. Figur 12. Das Federelement (50) schiebt nun den Kolbenbetätigungsstempel (76) nach unten, vgl. Figur 13. Mit der Abgabe des Medikaments über die Zylinder-Kolben-Einheit (100) ist der Injektionsvorgang beendet.

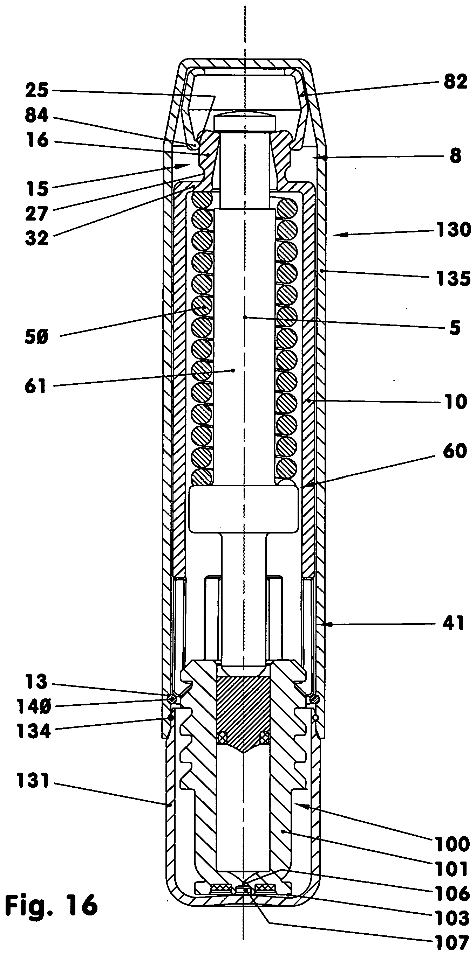

Die Figuren 16 bis 20 zeigen eine Einweginjektor-Variante, die vollständig in einem steril abgedichteten Umgehäuse (130) steckt. Das Umgehäuse (130) besteht aus einem großen Oberteil (135) und einem kleineren Unterteil (131). Es umgibt schützend den Einweginjektor.

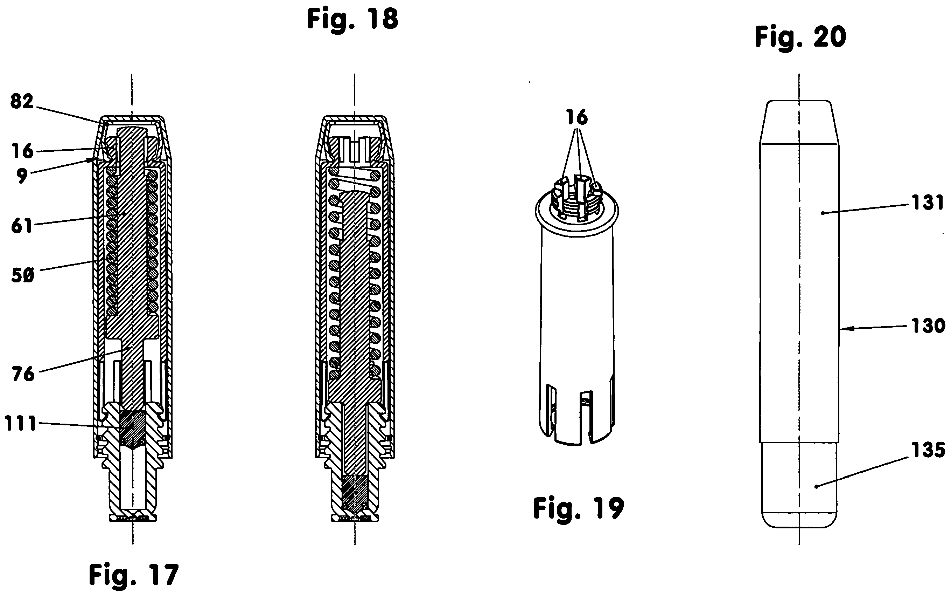

Das Oberteil (135) ist hier mit einer glatten, großteils zylindrischen Außenfläche dargestellt, vgl. auch Figur 20. Ggf. kann diese Außenfläche auch mit einer rutschfesten Struktur und/oder mit Griffschalen ausgestattet sein.

Beide Teile (131) und (135) sind steckbar miteinander verbunden. Dazu steckt nach Figur 16 das Unterteil (131) im Oberteil (135). Die Teile (131, 135) überlappen sich hierzu bereichsweise. In der Überlappungszone sitzt geschützt z.B. ein steril abdichtender Dichtring (134). Er ist z.B. mit dem Unterteil (131) verklebt.

Der Innenboden des Unterteils (131) ist im Bereich der Ausnehmung (107) z.B. mit einer Gummierung beschichtet, die sich bei der Montage des Umgehäuses (130) am Boden (103) der Zylinder-Kolben-Einheit (100) zumindest im mittleren Bodenbereich steril dichtend elastisch anschmiegt.

Im Umgehäuse (130) sitzen die aus den Figuren 5 bis 10 bekannten Einweginjektorteile (10, 50, 60 und 100). Auf den Sperrelementen (16) sitzt ein kappenförmiges Auslöseelement (82), das zumindest bezüglich seiner oberen Formgestaltung im Oberteil (135) eingepasst ist.

Das Gehäuse (10) ist im Oberteil (135) mittels eines z.B. torusförmigen Sicherungsringes (140) fixiert. Der Sicherungsring (140) ist in eine Gehäusenut (13) eingelassen.

Zur Montage dieses Einmalinjektors wird das Auslöseelement (82) über die Sperrelemente (16) gestülpt. Der Rand (84) befindet sich dabei im Rücksprungbereich (27), vgl. Figur 18. In einem weiteren Schritt wird das Federelement (50) auf den Kolbenbetätigungsstempel (60) gesetzt und mit diesem in das Gehäuse (10) von unten her z.B. bis zum Setzen des Federelements (50) hineingeschoben, vgl. Figur 17. Nun wird das Auslöseelement (82) nach oben gezogen, so dass der Rand (84) in die Kerbe (25) einrastet, vgl. auch Figur 6. Der Kolbenbetätigungsstempel (60) befindet sich jetzt in seiner Sperrstellung (8).

In einem weiteren Schritt wird die befüllte Zylinder-Kolben-Einheit (100) im Gehäuse (10) verrastet. Anschließend wird das Gehäuse (10) mit den bisher eingebauten Teilen (50, 60 und 100) in das Oberteil (135) eingeschoben und mittels des Sicherungsrings (140) gegen ein Herausfallen gesichert. Zum Abschluss der Montage wird das Unterteil (131) in das Oberteil (135) gesteckt.

Im Nutzungsfall wird der Einmalinjektor durch das Abziehen des Unterteils (131) entsichert. Zum Auslösen wird der Injektor am Oberteil (135) gehalten und mit der Düsenbohrung (106) voraus gegen die Hautpartie gepresst an der die Injektion erwünscht ist. Durch den Druck gegen die Haut schiebt das Oberteil (135) das Auslöseelement (82) gegen den Boden (32) des Gehäuses (10); der Injektionsvorgang beginnt. Nach dem raschen Entleeren des Zylinders (100) ist der Vorgang beendet.

Mit Ausnahme der Federelemente (50, 29) und der Kolbenplatte (116) sind alle Teile der zuvor beschriebenen Einweginjektoren aus Kunststoffen oder kunststoff- bzw. gummiähnlichen Werkstoffen gefertigt.

### Bezugszeichenliste:

- 1: Injektionslösung; Medikament
- 5: Mittellinie des Einweginjektors

- 8: Sperrstellung
- 9: Lösestellung

- 10: Gehäuse, einteilig
- 11: Gehäuseinnenraum
- 12: Gehäusestirnseite, unten
- 13: Gehäusenut
- 14: Ringnut
- 15: Auslösebereich
- 16: Sperrelemente
- 17: Hebelarm, kurz
- 18: Hebelarm, lang
- 19: Stützelemente

- 21: Anlagebereich
- 22: Anlagefläche, oben, innen
- 23: Anlagefläche, seitlich, außen
- 24: Haltebereich
- 25: Kerbe
- 27: Rücksprungbereich
- 28: Rücksprungkerbe, Rücksprungkontur
- 29: Schraubendruckfeder

- 31: Mantelbereich
- 32: Boden, Zwischenboden
- 34: Öffnung, Bohrung, Ausnehmung
- 35: Gehäusekragen
- 39: Gehäuseumgriff
- 41: Fixierbereich für die Zylinder-Kolben-Einheit,
- 42: Federhaken
- 43: Hakenspitze
- 44: Anfasung
- 45: Rillenprofil
- 46: Klemmhülse

- 50: Federelement, Schraubendruckfeder, Federenergiespeicher

- 60: Kolbenbetätigungsstempel
- 61: Zugstab
- 62: Führungszapfen
- 63: Abstützfläche, Torusteilfläche
- 64: Übergangsfläche

- 73: Stempelteller

- 76: Kolbenschieber

- 80: Auslöseeinheit
- 82: Auslöseelement
- 83: Boden, gerillt
- 84: Rand, nach innen gewölbt
- 85: Außenrand
- 86: Stützstege
- 87: Umgriffsrand
- 88: Steg, umlaufend
- 89: Rohrabschnitt
- 95: Sicherungselement
- 96: Scheibe, tellerförmig
- 97: Sperrzapfen
- 98: Griff

- 100: Zylinder-Kolben-Einheit
- 101: Zylinder
- 102: Rastrippen, außen; Außenrillung
- 103: Stirnfläche
- 106: Bohrung, Düse
- 107: Ausnehmung in der Stirnfläche
- 108: Erhebung

- 111: Kolben
- 112: Ringnut
- 114: Dichtring, Dichtung
- 116: Metallplatte, magnetisch oder magnetisierbar

- 120: Schutzfolie, Klebeversiegelung

- 130: Umgehäuse
- 131: Unterteil, Sicherungselement
- 132: Bohrung
- 133: Rand
- 134: Dichtung, steril
- 135: Oberteil, Sicherungselement
- 137: Rand
- 138: Umgriffe
- 139: Filmgelenk

- 140: Sicherungsring, torusförmig

## Patentansprüche

1. Einweginjektor mit einem Gehäuse (10), in dem oder an dem - jeweils zumindest bereichsweise - mindestens ein mechanischer Federenergiespeicher (50), mindestens eine - zumindest zeitweise wirkstoffbefüllbare - Zylinder-Kolben-Einheit (100), mindestens ein Kolbenbetätigungsstempel (60) und mindestens eine Auslöseeinheit (80) angeordnet ist, wobei der Federenergiespeicher (50) mindestens ein vorgespanntes Federelement umfasst, wobei zumindest ein Teil des Kolbenbetätigungsstempels (60) zwischen dem Federenergiespeicher (50) und dem Kolben (111) der Zylinder-Kolben-Einheit (100) positioniert ist und wobei der federbelastete Kolbenbetätigungsstempel (60) einen Zugstab (61) aufweist, der im Bereich seines hinteren Endes mindestens eine Abstützfläche (63) hat, **dadurch gekennzeichnet,**
- **dass** das Gehäuse (10) ein topfförmiger, unten offener Hohlkörper mit obenliegendem Zwischenboden (32) ist,
- **dass** an der oder den Abstützflächen (63) am Gehäuse (10) abgestützte Sperrelemente (16) anliegen, wobei die Sperrelemente (16) entweder Winkelhebel sind, die im Bereich ihrer Knickstelle am Zwischenboden (32) gelenkig gelagert sind oder wobei die Sperrelemente (16) an einem ringartigen, auf dem Zwischenboden (32) aufliegenden Bauteil befestigt sind oder wobei die Sperrelemente (16) als biegeelastische Elemente am Zwischenboden (32) angeformt sind, wobei die sperrende Lage der Sperrelemente (16) durch ein in einer Sperrstellung (8) positioniertes Auslöseelement (82) gesichert ist, und
- **dass** das Auslöseelement (82) eine Lösestellung (9) hat, die ein Freigeben der Sperrelemente (16) bewirkt.

2. Einweginjektor gemäß Anspruch 1, **dadurch gekennzeichnet, dass** das einzelne Sperrelement (16) einen Schwerpunkt hat, dessen kürzester Abstand zur Gehäusemittellinie (5) in der elastisch verformten Sperrstellung (8) kleiner ist als in der unverformten Lösestellung (9).

3. Einweginjektor gemäß Anspruch 1, **dadurch gekennzeichnet, dass** die Abstützfläche (63) die Innenfläche eines Torusmantels oder einer Kegelstumpfmantelfläche ist.

4. Einweginjektor gemäß Anspruch 3, **dadurch gekennzeichnet, dass** bei einer torusteilmantelförmigen Abstützfläche (63) die Abstützfläche (63) in einer Ebene konvex und zugleich in einer anderen Ebene konkav gekrümmt ist.

5. Einweginjektor gemäß Anspruch 1, **dadurch gekennzeichnet, dass** am Gehäuse (10, 130) oder an der Auslöseeinheit (80) ein Sicherungselement (95, 135) angeordnet ist, das den Zugstab (61) in einer Sperrstellung (8) sichert.

## Claims

1. Disposable injector comprising a housing (10) having arranged therein or thereon at least in portions thereof at least one mechanical spring-type energy storage device (50), at least one cylinder-piston unit (100) adapted to be filled with an active agent at least intermittently, at least one piston actuating plunger (60) and at least one release unit (80), said spring-type energy storage device (50) comprising at least one biased spring element with at least part of the piston actuating plunger (60) being positioned between the spring-type energy storage device (50) and the piston (111) of the cylinder-piston unit (100) and with the spring biased piston actuating plunger (60) including a pullrod (61) having in the area of its rear end at least one engagement surface (63), **characterized in that**
- the housing (10) constitutes a cup-shaped hollow body which is open at the lower end and has an intermediate bottom (32) therein, **in that**
- the engagement surface(s) (63) are engaged by locking elements (16) supported by the housing (10), with said locking elements (16) constituting angular levers movably mounted on the intermediate bottom (32) in the area of their bends or with the locking elements (16) secured to an annular component resting on the intermediate bottom (32) or with the locking elements being formed to be integral with the intermediate bottom (32) as resiliently flexing elements, with the locking position of the locking elements (16) secured by a release element (82) positioned in a locking position (8), and **in that**
- the release element (82) has a release position (9) in which it releases the locking elements (16).

2. Disposable injector as claimed in claim 1, **characterized in that** the single locking element (16) has a center of gravity of which the shortest distance to the center line (5) of the housing is shorter in the resiliently deformed locking position (8) than in the non-deformed release position (9).

3. Disposable injector as claimed in claim 1, **characterized in that** the engagement surface (63) is the inner surface of a mathematical toroid envelope or of the mathematical surface of a frustrum of a cone.

4. Disposable injector as claimed in claim 3, **characterized in that**, when the engagement surface (63) is a mathematical toroid partial envelope, said engagement surface (63) is convexly curved in one plane and at the same time concavely curved in another plane.

5. Disposable injector as claimed in claim 1, **characterized in that** the housing (10, 130) or the release unit (80) has located thereat a locking element (95, 135) which secures the pullrod (61) in a locked position.

## Revendications

1. Injecteur à usage unique comprenant un boîtier (10) dans lequel ou sur lequel sont disposés - à chaque fois au moins en partie - au moins un accumulateur d'énergie à ressort mécanique (50), au moins une unité cylindre-piston (100) - pouvant être remplie au moins temporairement d'une substance active, au moins un poinçon d'actionnement de piston (60) et au moins une unité de déclenchement (80), l'accumulateur d'énergie à ressort (50) comprenant au moins un élément à ressort précontraint, au moins une partie du poinçon d'actionnement de piston (60) étant positionnée entre l'accumulateur d'énergie à ressort (50) et le piston (111) de l'unité cylindre-piston (100) et le poinçon d'actionnement de piston (60) sollicité par la force d'un ressort présentant une tige de traction (61) qui possède au moins une surface d'appui (63) au niveau de son extrémité arrière,
**caractérisé en ce**
- **que** le boîtier (10) est un corps creux en forme de pot ouvert vers le bas avec fond intermédiaire supérieur (32)
- **que** des éléments de blocage (16) qui s'appuient sur le boîtier (10) reposent contre la ou les surfaces d'appui (63), les éléments de blocage (16) étant des leviers coudés fixés de manière articulée au niveau de leur point de flexion sur le fond intermédiaire (32) ou les éléments de blocage (16) étant fixés sur un composant de forme annulaire qui repose sur le fond intermédiaire (32) ou les éléments de blocage(16) étant façonnés sur le fond intermédiaire (32) comme éléments élastiques en flexion, la position de blocage des éléments de blocage (16) étant fixée par un élément de déclenchement (82) positionné dans une position de verrouillage (8) et
- **que** l'élément de déclenchement (82) présente une position de déblocage (9) qui provoque une libération des éléments de blocage (16).

2. Injecteur à usage unique selon la revendication 1, **caractérisé en ce que** chaque élément de blocage (16) a un centre de gravité dont la distance la plus courte par rapport à la ligne médiane du boîtier (5) dans la position de verrouillage (8) élastiquement déformée est inférieure à la distance dans la position de déblocage (9) non déformée.

3. Injecteur à usage unique selon la revendication 1, **caractérisé en ce que** la surface d'appui (63) est la surface intérieure d'une enveloppe d'un tore ou d'une enveloppe tronconique.

4. Injecteur à usage unique selon la revendication 3, **caractérisé en ce que**, dans le cas d'une surface d'appui (63) en forme d'enveloppe de tore, la surface d'appui (63) est incurvee de manière convexe dans un plan et incurvée de manière concave dans un autre plan.

5. Injecteur à usage unique selon la revendication 1, **caractérisé en ce qu'**un élément de sécurité (95, 135), qui fixe la tige de traction dans une position de verrouillage (8), est disposé sur le boîtier (10, 130) ou sur l'unité de déclenchement (80).
